(19) Europäisches Patentamt
European Patent Office
Office européen des brevets

(11) Veröffentlichungsnummer : **0 112 294 B1**

(12) **EUROPÄISCHE PATENTSCHRIFT**

(45) Veröffentlichungstag der Patentschrift : **30.09.87**

(51) Int. Cl.[4] : **C 07 D301/06**, C 07 D303/04, C 07 D303/08

(21) Anmeldenummer : **83810587.2**

(22) Anmeldetag : **14.12.83**

# (54) Photoinitiierte Epoxidierung von Allylchlorid zu Epichlorhydrin.

(30) Priorität : **20.12.82 US 450814**
**08.06.83 US 502384**

(43) Veröffentlichungstag der Anmeldung : **27.06.84 Patentblatt 84/26**

(45) Bekanntmachung des Hinweises auf die Patenterteilung : **30.09.87 Patentblatt 87/40**

(84) Benannte Vertragsstaaten : **CH DE FR GB LI**

(56) Entgegenhaltungen :
**US-A- 4 383 904**
**JOURNAL OF THE AMERICAN CHEMICAL SOCIETY, Band 98, Nr. 14, 7. Juli 1976, Seiten 4193-4200, Columbus, Ohio, USA, N. SHIMIZU et al.: "Photooxidation of olefins sensitized by alpha-diketones and by benzophenone. A practical epoxidation method with biacetyl"**
**CHEMICAL ABSTRACTS, Band 92, Nr. 20, 19. Mai 1980, Seite 16, Nr. 164507w, Columbus, Ohio, USA**
**JOURNAL OF THE AMERICAN CHEMICAL SOCIETY, Band 103, Nr. 8, 22. April 1981, Seiten 2049-2053, Columbus, Ohio, USA, Y. SAWAKI et al.: "Photoepoxidation of olefins with benzoins and oxygen. Epoxidation with acylperoxy radical"**
**JOURNAL OF ORGANIC CHEMISTRY, Band 48, Nr. 3, 11. Februar 1983, Seiten 337-342, Columbus, Ohio, USA , J.P. SHEPHERD: "Photoepoxidation of propylene at elevated pressures sensitized by alpha-diketones"**
**J.Am.chem.Soc. 91, 7554 (1969) und 95,7067 (1973)**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Mansfield, Kevin T.**
**80 Pinecrest Drive**
**North Kingstown, RI 02852 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 112 294 B1

Jouve, 18, rue St-Denis, 75001 Paris, France

**Beschreibung**

Die vorliegende Erfindung betrifft ein neues Verfahren zur photoinitiierten Epoxidierung (Photooxidation) von Allylchlorid zu Epichlorhydrin.

Durch Farbstoffe sensibilisierte Photooxidationen sind auf verschiedenen Gebieten bekannt. P. D. Barlett et al. [J. Am. Chem. Soc., 98, 4193 (1976) und Tetrahedron Lett., 2983 (1978)] stellten Untersuchungen über die Photooxidation von Arylolefinen, cycloaliphatischen und nichtcyclischen Olefinen an. Nach diesen Untersuchungen erfolgt zwar in Gegenwart von α-Diketon-Sensibilisatoren eine Epoxidierung ; dabei wird aber der Sensibilisator in manchen Fällen weitgehend oder vollständig zerstört.

Durch Farbstoffe sensibilisierte Photooxidationen von Olefinen wurden auch von C.S. Foote [Acc. Chem. Res., 1, 104 (1968)] untersucht. Unter den meisten Reaktionsbedingungen besteht danach das hauptsächliche Oxidationsprodukt von allylische Wasserstoffatome enthaltenden Olefinen aus den entsprechenden Hydroperoxiden. Von Barlett et al. wurde jedoch gefunden, dass die Photooxidation von hoch substituierten oder sterisch gehinderten Olefinen in Gegenwart von α-Diketonen häufig gute bis hohe Ausbeuten der entsprechenden Epoxide liefert.

T. Sasaki [J. Am. Chem. Soc., 103, 3882 (1981)] gelang es, Propylenoxid durch photochemische Oxidation des Propylens mit Sauerstoff in Acetonitril in Anwesenheit von Schwefeldioxid herzustellen. Die Reaktion scheint über einen Schwefeldioxid-Charge-Transfer-Komplex zu verlaufen, was in keiner Beziehung zur vorliegenden Erfindung steht, und Poly(propylensulfonat) entsteht, als eines der Hauptprodukte. Sasaki erwähnt auch spezifisch, dass « Propylenoxid unter den Reaktionsbedingungen nicht besonders stabil ist und dass es allmählich in Polymere umgewandelt wird ». Weiterhin stellt er fest, dass « Epoxidbildung in Lösungsmitteln, deren Ionisierungspotential unter ca. 9,5 liegt, und/oder deren dielektrische Konstante kleiner als ca. 10 ist, nicht beobachtet wurde ». Speziell wird erwähnt : « Propylenoxid kann nicht in Lösungsmitteln wie (unter anderen) Benzol... (und)... Dichlormethan erhalten werden ».

Y. Sawaki und Y. Ogata [J. Am. Chem. Soc., 103, 2049 (1981)] beschreiben die Photoepoxidierung von Olefinen mit α-Acyloinen und Sauerstoff. Die Acyloin-Sensibilisatoren werden allerdings dabei fast vollständig zerstört.

Epichlorhydrin ist ein wichtiges Ausgangsmaterial bei der Grossherstellung von Komponenten für Epoxidharze. Bei den zur Zeit üblichen industriellen Verfahren zur Herstellung von Epichlorhydrin werden relativ starke Oxidationsmittel, wie Hydroperoxide, Hypohalogenite und dergleichen benötigt. Die Verwendung solcher Oxidationsmittel erfordert für eine sichere Handhabung und die Wiedergewinnung des Oxidationsmittels sowie auch für die aufwendige Aufarbeitung der dabei entstehenden Nebeprodukte eine kostspielige Technologie.

Die Möglichkeit einer direkten Oxidation unter Verwendung von Luft oder Sauerstoff als Sauerstoffquelle wie auch die Verwendung direkter Oxidationsmittel wäre für die industrielle Herstellung von Epichlorhydrin von grosser Bedeutung, besonders auch in wirtschaftlicher Hinsicht.

Aufgabe der Erfindung ist die Bereitstellung eines Verfahrens für die direkte photoinitiierte Epoxidierung von Allylchlorid zu Epichlorhydrin. Dieses Ziel sowie weitere damit verbundene Vorteile werden durch das unten beschriebene erfindungsgemässe Verfahren erreicht.

Gegenstand der Erfindung ist somit insbesondere ein neues Verfahren zur Herstellung von Epichlorhydrin durch Epoxidierung von Allylchlorid das dadurch gekennzeichnet ist, dass man eine mit Sauerstoff gesättigte Lösung von Allylchlorid in einem aprotischen Lösungsmittel in Gegenwart einer zur Epoxidierung des Allylchlorids zum Epichlorhydrin ausreichenden Menge eines α-Diketon-Sensibilisators mit einer 450 Watt Mitteldruckquecksilberlampe bestrahlt.

Geeignete α-Diketon-Sensibilisatoren sind z. B. Biacetyl (2,3-Butandion), Benzil (Diphenylethandion), oder 1-Phenyl-1,2-propandion. Es können auch Gemische verschiedener Sensibilisatoren verwendet werden. Andere α-Diketone, die im gleichen UV- und sichtbaren Bereich des Spektrums absorbieren, sollten erwartungsgemäss auch geeignet sein. Andere übliche Sensibilisatoren für Photooxidationen, wie die Farbstoffe Methylenblau, Bengalrot B, Tetraphenylporphin und polymergebundenes Rose Bengal, sowie auch Benzophenon, bewirken jedoch die gewünschte Epoxidierung nicht. Die bevorzugten Sensibilisatoren sind Biacetyl oder Benzil.

Der α-Diketon-Sensibilisator wird erfindungsgemäss in einer Menge eingesetzt, welche die Epoxidierung unter den erfindungsgemässen Verfahrensbedingungen bewirkt. Im allgemeinen werden im wesentlichen äquimolare Mengen des α-Diketon-Sensibilisators, bezogen auf das zu epoxidierende Allylchlorid eingesetzt ; es können jedoch mit zufriedenstellenden Resultaten auch 0,3 bis 2 Mol-Aequivalente α-Diketon-Sensibilisator pro Mol Allylchlorid verwendet werden.

Erfindungsgemäss geeignete aprotische Lösungsmittel sind z. B. aliphatische Kohlenwasserstoffe, wie die verschiedenen Pentane, Hexane, Cyclohexan, Heptane, Methylcyclohexan, Octane usw. aromatische Kohlenwasserstoffe, wie Benzol, Toluol, Ethylbenzol, Xylol und deren verschiedene chlorierte Derivate ; chlorierte aliphatische Kohlenwasserstoffe, wie Methylenchlorid, Chloroform und Tetrachlorkohlenstoff ; Ether, wie Diethylether, Tetrahydrofuran und Dioxan ; Ester, wie Ethylacetat, Nitrile, wie Acetonitril und Propionitril und weitere aprotische Lösungsmittel wie z. B. Nitromethan.

Besonders geeignete aprotische Lösungsmittel für die Durchführung des erfindungsgemässen Verfahrens sind Benzol und Methylenchlorid. Methylenchlorid ist bei der Herstellung von Epichlorhydrin aus Allylchlorid besonders bevorzugt, weil diese Epoxidierungsreaktion in Methylenchlorid selektiver als in Benzol verläuft.

Die Bestrahlung erfolgt bei Wellenlängen, wie sie mit einer 450 Watt Mitteldruckquecksilberlampe erzielt werden. Die mit Sauerstoff gesättigten Lösungen des Olefins in Benzol oder Methylenchlorid werden zweckmässig auf Temperaturen bis zu 30 °C, und besonders zwischen 20 und 26 °C gehalten, dabei wird das Reaktionsgemisch bevorzugt kontinuierlich mit gasförmigem Sauerstoff gesättigt. Als Quelle für den gasförmigen Sauerstoff wird mit Vorteil Druckluft verwendet.

Obwohl die Reaktion im Laboratorium geeigneterweise bei etwa atmosphärischem Druck durchgeführt wird, kann man bei erhöhtem Druck, zur Erhöhung der Löslichkeit der gasförmigen Reaktanten im Lösungsmittel höhere Reaktionsgeschwindigkeiten erwarten.

Gemäss einer bevorzugten Ausführungsform wird die Photooxidation in einem mit einem Mantel versehenen Photolyse-Gerät durchgeführt. Als Bestrahlungsquelle wird bevorzugt mindestens eine 450 Watt Mitteldruckquecksilberlampe, z. B. des Typs « Hanovia » (Fa. Hanovia Lamp Division, Newark, N.J.) verwendet. Mittels eines Pyrex-Schildes wird Licht mit einer Wellenlänge unter 318 nm, das unerwünschte Nebenreaktionen verursacht, wegfiltriert.

650 Watt Wolfram-Iod-Lampen, wie die von General Electric (DWY) hergestellten Lampen sowie eine handelsübliche « GE Sunlamp » stellen im Gegensatz zu den erfindungsgemäss einzusetzenden 450 Watt Mitteldruckquecksilberlampen keine befriedigenden Bestrahlungsquellen dar. Sämtliche Versuche, Olefine mit solchen Lampen zu epoxidieren, waren erfolglos.

Die Photooxidation des Allylchlorids zum Epichlorhydrin läuft nach dem folgenden Schema ab :

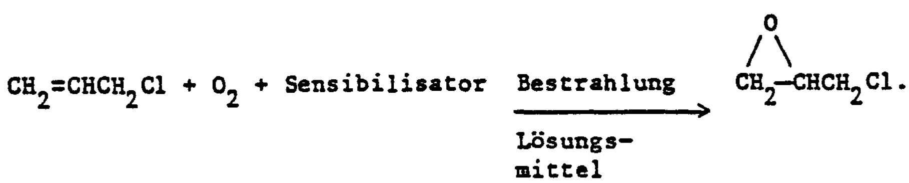


Die folgenden Beispiele veranschaulichen das erfindungsgemässe Verfahren. Bei den verwendeten α-Diketon-Sensibilisatoren und Olefinen handelt es sich um käufliche Produkte.

Beispiel 1 : Herstellung von Epichlorhydrin

Eine Lösung von 11,3 g (0,148 Mol) Allylchlorid und 12,6 g (0,147 Mol) Biacetyl in 400 ml Benzol (oder Methylenchlorid) wird in einem 700 ml Photolyse-Gerät vorgelegt, das mit einem Wasserkühlmantel, und einer von einem Pyrex-Schild umgebenen 450 Watt Mitteldruckquecksilberlampe « Hanovia » als Bestrahlungsquelle ausgerüstet ist. Nach dem Einschalten der Wasserkühlung im Mantel wird Sauerstoff durch ein mit Glasfritte versehenens Glasrohr unter kräftigem Rühren durch das Reaktionsgemisch geblasen. Die Lösung wird mit der Quecksilberhochdrucklampe bestrahlt. Dabei wird die Temperatur des Reaktionsgemisches zwischen 20 und 26 °C gehalten.

Der Verlauf der Reaktion wird durch periodische Entnahme von Proben des Reaktionsgemisches zwecks Untersuchung mittels Gaschromatographie verfolgt. Diese Proben des Reaktionsgemisches reagieren beim Stärke-Iod-Test im allgemeinen positiv, was auf die Anwesenheit von Allylhydroperoxiden hindeutet.

In Tabelle 1 sind die Resultate der Versuche 1a) bis 1g) mit unterschiedlichen Bestrahlungszeiten, Lösungsmitteln und Sensibilisatoren angegeben.

(Siehe Tabelle Seite 4 f.)

Tabelle 1 : Photochemische Oxidation von Allylchlorid zu Epichlorhydrin (EPI) unter Verwendung einer Mitteldruckquecksilberlampe.

| Versuch | Sensibilisator | Lösungsmittel | Bestrahlungszeit | GC-Analyse Allylchlorid % | GC-Analyse EPI % | nicht identifizierte Produkte % | geschätzte Ausbeute an EPI % |
|---|---|---|---|---|---|---|---|
| 1a) | Benzil | $CH_2Cl_2$ | 0 Min. | 6,70 | nicht feststellbar | 0,40 | 0,0 |
| 1b) | Benzil | $CH_2Cl_2$ | 15 Min. | 6,60 | nicht feststellbar | 0,40 | 0,0 |
| 1c) | Benzil | $CH_2Cl_2$ | 55 Min. | 6,40 | 0,03 | 0,41 | 0,5 |
| 1d) | Biacetyl | Benzol | 35 Min. | 1,00 | 0,013 | 0,02 | 1,3 |
| 1e) | Biacetyl | Benzol | 1 h 15 Min. | 0,85 | 0,017 | 0,24 | 1,7 |
| 1f) | Biacetyl | Benzol | 4 h 5 Min. | 0,34 | 0,027 | 0,40 | 2,7 |
| 1g) | Benzil | Benzol | 1 h 0 Min. | 0,20 | 0,010 | 0,08 | 0,8 |
| 1h) | Benzil | Benzol | 6 h 45 Min. | 1,00 | 0,032 | 0,15 | 2,7 |
| 1i)[2] | Biacetal | Benzol | 1 h 45 Min. | 37,30 | 0,05 | 0,08 | 0,1 |
| 1j)[3] | Biacetyl | Benzol | 2 h 45 Min. | 32,10 | 0,05 | 2,10 | 0,1 |

[1] GC-Analysen sind in Flächenprozent der Signale angegeben

[2] Gewichtsverhältnis Allylchlorid/Benzol = 2,45 ; 6 Mol.% Biacetyl verwendet

[3] Der Probe i) wird Essigsäure zugegeben, dann wird die Bestrahlung im Versuch j) weitergeführt, um Peressigsäure in situ zu bilden.

Die Gaschromatographische Analyse (% der Signaloberfläche) ergibt, dass die Ausbeute an Epichlorhydrin etwa 3 % ist, wobei auch mehrere unbekannte Verbindungen entstehen. In Methylenchlorid als Lösungsmittel kommt es zu einer saubereren Umsetzung und die Ausbeute an Epichlorhydrin in einer Stunde ist 0,5 bis 1,0 % mit nur zwei unbekannten Nebenprodukten.

Das erfindungsgemässe Verfahren ist hier anhand bevorzugter Ausführungsformen beschrieben. Uebliche Variationen in Materialien und Geräten liegen jedoch innerhalb des Umfangs der beanspruchten Erfindung, wie sie in den Ansprüchen beschrieben ist.

**Patentansprüche**

1. Verfahren zur Herstellung von Epichlorhydrin mittels Epoxidierung von Allylchlorid, dadurch gekennzeichnet, dass man eine mit Sauerstoff gesättigte Lösung von Allylchlorid in einem aprotischen Lösungsmittel in Gegenwart einer zur Epoxidierung des Allylchlorids zum Epichlorhydrin ausreichenden Menge eines α-Diketon-Sensibilisators mit einer 450 Watt Mitteldruckquecksilberlampe bestrahlt.

2. Verfahren nach Anspruch 1, dadurch gekennzeichnet, dass man als α-Diketon-Sensibilisator Biacetyl, Benzil oder 1-Phenyl-1,2-propandion verwendet.

3. Verfahren nach Anspruch 1, dadurch gekennzeichnet, dass man als α-Diketon-Sensibilisator Biacetyl oder Benzil verwendet.

4. Verfahren nach Anspruch 1, dadurch gekennzeichnet, dass man als aprotisches Lösungsmittel Benzol oder Methylenchlorid verwendet.

5. Verfahren nach Anspruch 1, dadurch gekennzeichnet, dass man als aprotisches Lösungsmittel Methylenchlorid verwendet.

**Claims**

1. A process for the preparation of epichlorohydrin by the epoxidation of allyl chloride, which comprises irradiating an oxygen-saturated solution of allyl chloride with a 450 watt medium-pressure mercury lamp in an aprotic solvent and in the presence of an amount of an α-diketone sensitizer sufficient to effect epoxidation of the allyl chloride to epichlorohydrin.

2. A process according to claim 1, wherein the α-diketone sensitizer is biacetyl, benzil or 1-phenyl-1,2-propanedione.

3. A process according to claim 1, wherein the α-diketone sensitizer is biacetyl or benzil.

4. A process according to claim 1, wherein the aprotic solvent is benzene or methylene chloride.

5. A process according to claim 1, wherein the aprotic solvent is methylene chloride.

**Revendications**

1. Procédé pour préparer l'épichlorhydrine par époxydation du chlorure d'allyle, procédé caractérisé en ce qu'on irradie au moyen d'une lampe à mercure moyenne pression d'une puissance de 450 W une solution du chlorure d'allyle dans un solvant aprotique, saturée d'oxygène, en présence d'une quantité d'un sensibilisateur α-dicétonique suffisante pour provoquer la conversion du chlorure d'allyle en épichlorhydrine par époxydation.

2. Procédé selon la revendication 1 caractérisé en ce qu'on utilise, comme sensibilisateur α-dicétonique, le diacétyle, le benzile ou la phényl-1 propane-dione-1,2.

3. Procédé selon la revendication 1 caractérisé en ce qu'on utilise, comme sensibilisateur α-dicétonique, le diacétyle ou le benzile.

4. Procédé selon la revendication 1 caractérisé en ce qu'on utilise, comme solvant aprotique, le benzène ou le chlorure de méthylène.

5. Procédé selon la revendication 1 caractérisé en ce qu'on utilise le chlorure de méthylène comme solvant aprotique.